# EUROPEAN PATENT APPLICATION

(11) **EP 1 941 850 A2**
(43) Date of publication of application: **09.07.2008**
(21) Application number: 07021269.1
(22) Date of filing: 31.10.2007
(51) Int. Cl.: A61F 9/02, G02C 7/16

(54) **Personal safety device**

(30) Priority: 07.11.2006 SE 0602365
(71) Applicant: Boljanac, Bertil, 302 38 Halmstad (SE)
(72) Inventor: Boljanac, Bertil, 302 38 Halmstad (SE)
(74) Representative: Wallengren, Yngvar

(57) **Abstract**

Safety glasses (1) which are produced in one piece from a sheet- or foil material of plastic. The safety glasses (1) have a bending for realising a spring action which is sufficient for fixing and retaining the safety glasses on a wearer's head. The safety glasses (1) are of one piece manufacture and are separable and rolled up together on a roll, the arching direction of the roll coinciding with the bending direction of the safety glasses (1).

A method of producing safety glasses (1) comprises the steps that the outer contours of the safety glasses (1) are formed from a web of sheet- or foil material. In one step, each one of the safety glasses (1) is given a permanent bending for realising a spring action. The inherent spring action is sufficient for fixing and retaining the safety glasses on a wearer's head. The bending is realised by heating of the safety glasses (1), whereafter they are cooled for realising a permanent bending.

## Description

### TECHNICAL FIELD

The present invention relates to a personal safety device.

The present invention also relates to a method of producing personal safety devices.

### BACKGROUND ART

There are innumerable different safety devices are available for sale on the market, such as, for example, breathing protection, safety glasses etc. There is a plurality of various safety glasses that comprise a frame with lenses which are often produced entirely from a hard plastic material. Such safety goggles or glasses are occasionally employed in hobby activities, but above all are used by professionals at, for example, building sites, within the manufacturing industry, etc.

One problem that arises with safety glasses that are intended for repeated use is that they are difficult to store and often become scratched and dusty on use, or as a result of incorrect storage between periods of use, for example in a toolbox full of tools. A problem which then thus arises is that the user is disinclined to wear scratched safety glasses and is often not motivated to collect a new pair of safety glasses. The result will be that work is carried out without the use of safety glasses, which is not to be recommended from the point of view of the working environment and risk of personal working injury.

Safety glasses which are intended for repeated use are also relatively expensive to purchase and, given that the wearer seldom uses a protective case for storing the glasses, but places them quite simply among other tools in the toolbox, this implies that safety glasses of this type will not achieve a particularly lengthy service life. Hence, a considerable number of safety glasses are scrapped beforehand and this entails a considerable cost for, for example, the construction company when purchasing new safety equipment.

Breathing protection available on the market is often also intended for repeated use, and corresponding problems to those involving safety glasses occur in the periods between use. Breathing protection which is not stored correctly in these inactive periods runs the risk of becoming dirty or damaged to the extent that they are no longer usable. In addition to increased costs, there is also a risk that some work be carried out without the use of breathing protection apparatus, even though this would genuinely be needed.

### PROBLEM STRUCTURE

The present invention has for its object to design the safety device intimated by way of introduction so that it obviates the drawbacks in the prior art technology. In particular, the present invention has for its object to realise safety devices which are economical to manufacture and are intended for single use. Finally, the present invention also for its object to realise safety devices which can be stored readily accessibly in a manner that requires little space, until needed for use.

### SOLUTION

The objects forming the basis of the present invention will be attained if the personal safety device intimated by way of introduction is characterised in that safety units are separably united to one another in an elongate strip or band and are disposed on a storage unit.

The objects of the present invention will also be attained in respect of the method intimated by way of introduction, if this is characterised in that the safety unit is manufactured in the form of a strip or band which is thereafter disposed on a storage unit.

### BRIEF DESCRIPTION OF THE ACCOMPANYING DRAWINGS

The present invention will now be described in greater detail hereinbelow, with reference to the accompanying Drawings. In the accompanying Drawings:
- Fig. 1: is a straight front elevation of safety glasses in a flat state;
- Fig. 2: is a straight front elevation of the safety glasses in a bent state; and
- Fig. 3: is an isometric view of the production of safety glasses.

### DESCRIPTION OF PREFERRED EMBODIMENT

In the description given below, directional and positional disclosures will be used such as, for example, inwards, outwards, upwards, downwards, etc. These relate to a situation which is normal for the use of the device according to the present invention, on fixing and retaining them on a wearer's head.

The subject matter of the present invention relates to a personal safety device. The safety device comprises safety units which are combined to one another in a preferably elongate strip. The safety units are separable from one another and applied or disposed on a storage unit. The storage unit may, for instance, be in the form of a roll or a core. The safety devices consist, for example, of safety glasses or breathing protection.

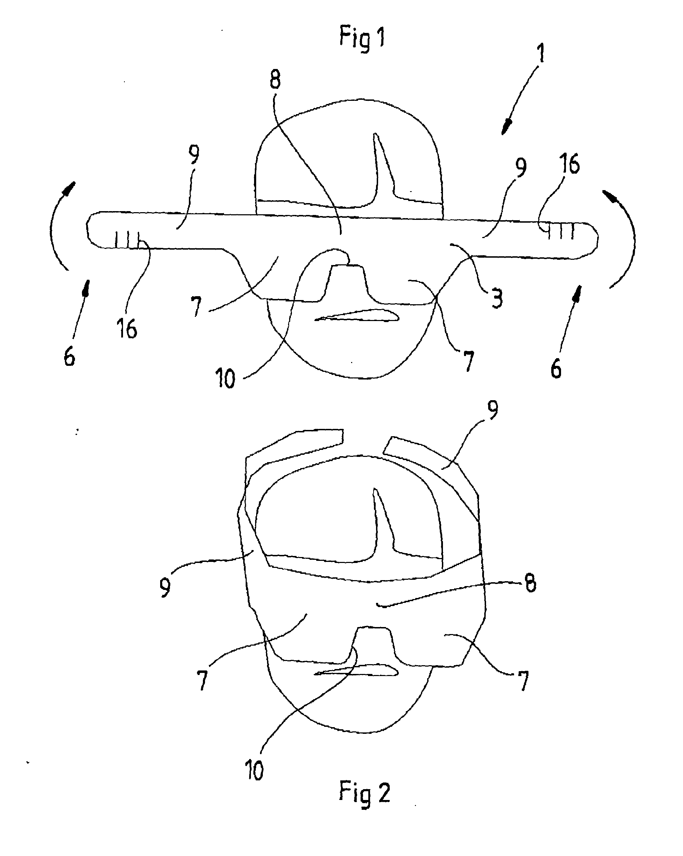

Fig. 1 shows a pair of safety glasses 1 in a flat state, the glasses being produced from a sheet- or foil material 2. The sheet- or foil material is a transparent material, of, for example, plastic. The safety glasses 1 are intended to be used once, for instance during one working day, and then be scrapped. The safety glasses 1 are prestressed in order to realise a springing resilient function, sufficient for fixing and retaining them on a wearer's head when being used, as shown in Fig. 2. The prestressing in the safety glasses is preferably realised by a bending of the sheet- or foil material 2. As regards the thickness of the material, this must be sufficient in order to ensure the necessary mechanical strength of the safety glasses, but should not be so large that the flexing or bending operation at working temperatures is impeded or rendered impossible. In addition, the material displays good elastic or resilient properties, so that it may be flexed and be bent at the working temperatures without suffering from any permanent deformation. A suitable thickness may be in the range of between 0.5 and 1.0 mm.

A plurality of safety glasses are disposed in one piece 3 and are separable and rolled up on a roll 4 for facilitating their access at, for example, a building site or in a hobby room.

The direction of arching of the roll 4 coincides with the direction of bending of the safety glasses 1. The safety glasses 1 have weakened portions 5 disposed in their free ends 6, for simple separation from one another. The weakened portions 5 may, for example, consist of perforations in the sheet- or foil material 2.

The safety glasses 1 have viewing panels 7 through which the wearer can see. On the viewing panels 7, a safety film is disposed for protecting the viewing panels 7 on storage, for example on the roll 4. The safety film has a lightly adhesive property for adhesion to the viewing panels 7, but the safety film is very easy to tear off before use.

The viewing panels 7 of the safety glasses 1 are connected to a support bridge 8 which, on use, is placed on the wearer's nose. On the sides of the viewing panels 7, there are elongate side portions 9 disposed at the free ends 6, in the form of bows or shafts for support and contact against the head of the wearer. The support bridge 8 for the nose consists of a recess 10 between the viewing panels 7 in the sheet- or foil material 2.

As an extra provision to ensure the retention of the safety glasses 1 on the wearer's head, slots 16 may be provided in the side portions 9. The slots 16 may, for example, in the one side portion 9, be disposed to extend from the lower edge of the side portion 9 and, in the other side portion 9, to extend from its upper edge. The slots 16 extend preferably half way through the side portions 9, Thus, on use the slots 16 may be hooked into one another behind the wearer's head.

In another modified embodiment, the safety glasses 1 may be disposed on a carrier, in the form of a foil. Instead of the safety glasses 1 having weakened portions 5, the carrier has weakened portions that are disposed between the free ends of the safety glasses.

The above-considered bending or flexing applies above all to the side portions 9 since they must fit tightly around the wearer's head in order for the safety glasses to remain reliably in position. However, the viewing panels 7, and preferably also the support bridge 8 are provided with a bend to ensure that the distance between the viewing panels 7 and the eyes of the wearer is large enough that the wearer can blink unimpeded. In such instance the bending of the viewing panels 7 and the support bridge may be different, preferably smaller, than the bending in the side portions 9.

The subject matter of the present invention also relates to a method for the production of the personal safety device in which safety units are manufactured in the form of a strip or band which is thereafter applied on a storage unit. The storage unit consists, for example, of a roll 4 on which the safety units 1 are rolled up. In the method according to the invention, for example safety glasses or breathing protection are produced. In the production of the breathing protection devices, the outer contours thereof are formed from the strip, from a web of filter paper.

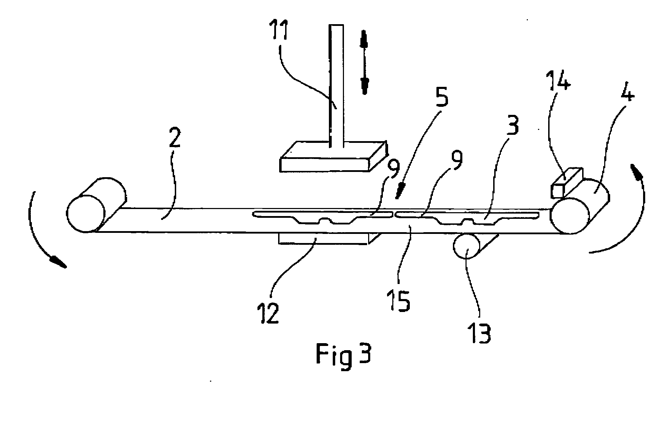

Fig. 3 shows a method for the production of the safety glasses 1 in accordance with the foregoing. The method comprises the steps that outer contours of the safety glasses 1 are formed from a web of sheet- or foil material 2 of, for example, plastic. The web with the sheet- or foil material 2 may also be rolled up on the roll 4. The forming takes place preferably by punching or by other requisite means. The punching operation may be put into effect with the aid of two rotary punching tools between which the sheet- or foil material is fed, or by a vertically reciprocal punching tool 11 with a die plate 12, as shown in Fig. 3. In the punching operation, slots 16 may also be formed in the side portions 9.

The web of sheet- or foil material 2 may be of a width which is sufficient for the production of a row of safety glasses 1 in sequence after one another. In another case, the web may display a width which is sufficient for the production of two or more rows of safety glasses 1, disposed in side-by-side relationship. With this latter variation, material waste in production may be reduced in that, for example, two rows of safety glasses are punched out alternatingly. Fig. 3 shows a row of safety glasses which are disposed with their free ends towards one another. If a second row were to be punched out in the same material web, for example one viewing panel 7 on a pair of glasses could be disposed in a space 15 opposingly formed along the side portions 9 of two safety glasses, and so on along the entire web.

Each one of the safety glasses is given a permanent bending for realising a spring action which is sufficient for fixing and retaining the safety glasses on a wearer's head.

In order to realise the bending, use is made of a heating device 13 by means of which the safety glasses 1 are heated until plasticity is reached in the material, e.g. from a hot air current or by other requisite means. After the heating, the safety glasses 1 are cooled with the aid of a cooling device 14 in order to assume a permanent bending in the material. The cooling may also be put into effect by a cooling air current, or by other appropriate means.

After the heating of the safety glasses 1, they may pass through a forming station, in which the viewing panels 7 are given smaller arching than the elongate side portions 9 for realising a larger distance between the eyes of the wearer and the viewing panels 7.

After the heating of the safety glasses 1, they can be directly rolled up on the roll 4 in order subsequently to cool down on the roll and obtain their bend. In this case, no cooling apparatus is thus required.

After production, the safety glasses 1 are rolled up on a roll 4. On rolling onto the roll 4, the safety glasses 1 are arched in a direction that coincides with the bending direction, as the safety glasses 1 are worn by the user. The rolling up of the safety glasses 1 on the roll 4 helps the safety glasses to maintain their bent shape also during storage.

Before or during rolling up of the safety glasses 1 on the roll 4, excess material is preferably removed, in which event only the safety glasses 1 are rolled up on the roll 4.

### DESCRIPTION OF ALTERNATIVE EMBODIMENTS

Prestressing in the safety glasses 1 may also be realised with the aid of, for example, a memory material which is applied in the upper edge of the safety glasses. The memory material is preferably a memory metal which is applied on the safety glasses with the aid of a double-sided adhesive tape. The memory material may also be enclosed in the sheet- or foil material. The memory metal affords to the safety glasses their prestressing. The prestressing in the safety glasses 1 may also be realised with the aid of a shrink-on tape which is applied in the upper edge of the viewing panels 7. The shrink-on tape realises a bending of the safety glasses.

Further, the bending in the sheet- or foil material 2 may be preformed in the strip or band material before the production of the safety glasses 1 is commenced.

The bending in the sheet- or foil material 2 may also be realised in the material in that the sheet- or foil material passes and is drawn over a sharp edge.

After the forming of the safety device, this may be rolled up onto a roll 4, whereafter the safety device is lifted off from the roll 4. In this case, the safety device has a bending which is large enough to realise a "self-supporting" and self-winding roll 4.

In one alternative embodiment, the weakened portions 5 in the safety glasses may be disposed in the upper edge and lower edge of the viewing panels 7.

## Claims

1. Safety glasses, **characterised in that** the safety glasses are separably united with one another in an elongate strip or band (1) and are disposed on a storage unit.

2. The safety glasses as claimed in Claim 1, **characterised in that** the storage unit consists of a roll (4).

3. The safety glasses as claimed in Claim 1 or 2, **characterised in that** the safety glasses (1) are produced from a sheet- or foil material (2) in, e.g. plastic and have a prestressing which is sufficient for fixing and retaining of the safety glasses (1) on a wearer's head.

4. The safety glasses as claimed in Claim 3, **characterised in that** the prestressing in the safety glasses (1) is realised by a bending of the sheet- or foil material (2).

5. The safety glasses as claimed in Claim 2 and 4, **characterised in that** the bending direction of the safety glasses (1) coincides with the arching direction of the roll (4).

6. The safety glasses as claimed in Claims 1 to 5, **characterised in that** the safety glasses (1) have weakened portions (5) disposed in their free ends (6), for simple separation from one another.

7. The safety glasses as claimed in Claim 6, **characterised in that**, in the region of the free ends (6), slots are provided for catching in one another behind a wearer's head, for ensuring the retention of the safety glasses (1).

8. A method of producing safety glasses, **characterised in that** the safety glasses are manufactured in the form of a strip or band which is thereafter disposed on a storage imit.

9. The method as claimed in Claim 8, **characterised in that** the safety glasses are punched out from the strip.

10. The method as claimed in Claim 8 or 9, **characterised in that** the application disposition comprises rolling up the safety glasses on a roll (4).

11. The method as claimed in Claim 8, **characterised in that** outer contours of the safety glasses (1) are formed from the strip of transparent sheet- or foil material (2), e.g. plastic, each of the safety glasses (1) being given a permanent bending for realising a resilient spring action sufficient for fixing and retaining the safety glasses (1) on a wearer's head.

12. The method as claimed in Claim 10 and 11, **characterised in that** the safety glasses (1), on rolling up, are arched in a direction which coincides with the bending direction.

13. The method as claimed in Claim 11 or 12, **characterised in that** the safety glasses (1) are heated for realising the bending.

14. The method as claimed in any of Claims 11 to 13, **characterised in that** the safety glasses (1) are cooled for realising a permanent bending.
